# EUROPEAN PATENT APPLICATION

(11) **EP 4 273 181 A1**
(43) Date of publication of application: **08.11.2023**
(21) Application number: 21915912.6
(22) Date of filing: 28.12.2021
(51) Int. Cl.: C08F 220/22, C08F 8/42, C08F 8/32, G01N 27/327, C12Q 1/00, C12Q 1/26

(54) **POLYMER COMPRISING PENTAFLUOROPHENYL ESTER AND ELECTROCHEMICAL BIOSENSOR COMPRISING SAME**

(30) Priority: 31.12.2020 KR 20200189140
(71) Applicant: I-SENS, INC., Seoul 06646 (KR); Sogang University Research Business Development Foundation, Seoul 04107 (KR)
(72) Inventor: SHIN, Hyunseo, Seoul 04138 (KR); MOON, Bongjin, Goyang-si, Gyeonggi-do 10416 (KR); KWON, Hoejun, Seoul 04745 (KR)
(74) Representative: Arnold & Siedsma
(86) International application number: PCT/KR2021/095143
(87) International publication number: WO 2022/146115

(57) **Abstract**

The present invention relates to a polypentafluorophenyl ester-based polymer used in the manufacturing of an electrochemical sensor, wherein the polymer has high reactivity with an amine functional group, causes less hydrolysis, retains good solubility, for general purposes, in organic solvents, and has low steric hindrance and toxicity or side effects compared with polymers having a similar structure, and thus is useful in an insertion-type device, especially a continuous glucose monitoring system, a part of which is inserted into the human body.

## Description

### [TECHNICAL FIELD]

### Cross-reference to related application(s)

The present application claims the priority based on Korean Patent Application No. 10-2020-0189140 filed on December 31, 2020, and all the contents disclosed in the document of the corresponding Korean Patent Application are incorporated by reference as a part of the present description.

The present invention relates to a polymer comprising pentafluorophenyl ester useful for fixing an electron transfer mediator and oxidoreductase on an electrode surface in a device such as an electrochemical biosensor and an electrochemical biosensor comprising thereof.

### [BACKGROUND ART]

Diabetes is a disease that occurs when high blood glucose levels are maintained for a long time and causes complications such as cardiovascular disease, stroke, kidney disease, and the like. In response to the high blood glucose level, it is necessary to reduce the blood glucose level through insulin injection, but if insulin is over-injected, hypoglycemia may occur, which can lead to shock or death. To prevent this, it is essential for diabetic patients to continuously measure the blood glucose concentration in the body using a blood glucose sensor for the purpose of maintaining an appropriate blood glucose level.

Recently, a blood glucose sensor to which a continuous glucose monitoring system (CGMS) is applied has been studied and commercialized. The CGMS sensor is a device that is inserted into subcutaneous tissue and continuously measures the glucose concentration through intercellular fluid, not blood. In case of a finger blood gathering method, it is difficult to check the exact change in the blood glucose level by measuring blood glucose 5 to 6 times a day, whereas the CGMS has an advantage of checking the change and tendency of the blood glucose level in a day. Through this, patients can quickly recognize hyperglycemia or hypoglycemia, and the ability to manage blood glucose can be further improved.

Biosensor refers to a device that selectively detects a biological sample and converts it into a specific signal. In particular, an enzyme-based biosensor using an electrochemical method is preferred because of its improved selectivity, miniaturization and measurement accuracy. A blood glucose biosensor using the electrochemical method is largely divided into first-generation and second-generation methods. The first-generation blood glucose sensor was first developed by Clark and Lyon, and is a method of measuring a blood glucose level through a decrease in oxygen concentration and a change in concentration of hydrogen peroxide generated, through an enzyme oxidation-reduction reaction of an enzyme, and the second-generation blood glucose sensor is a method of transferring electrons generated through an oxidation-reduction reaction of an enzyme to an electrode through an electron transfer mediator. The second-generation sensor has many advantages compared to the first-generation sensor in that there is less error depending on the oxygen concentration and the electron transfer reaction by a mediator is efficient and fast. For this reason, the second-generation sensor method comprising an electron transfer mediator is being applied to a CGMS blood glucose sensor.

An enzyme-based blood glucose biosensor of the electrochemical method is generally composed of an enzyme, an electron transfer mediator and an electrode. At first, glucose is oxidized to gluconolactone by an enzyme and then the reduced enzyme is oxidized and donates electrons to the electron transfer mediator. Then, the reduced medium is oxidized and passes through a process of transferring electrons to the electrode. From this series of processes, the blood glucose level can be confirmed as an electrical signal.

In production of a blood glucose sensor, the method of immobilizing an electron transfer mediator and an enzyme on an electrode surface is very important. Until now, methods for immobilizing an oxidation-reduction hydrating polymer comprising an electron transfer mediator on an electrode by mixing with an enzyme and adding a crosslinking agent have been studied. Among them, polyvinylpyridine and polyvinylimidazole are well known as an oxidation-reduction polymer matrix. However, these conventional oxidation-reduction polymers have problems in that a synthesis step of a final material is long and complicated, and low immobilization efficiency of a transition metal complex is exhibited, and also introduction of other functionalities into the polymer is difficult. Therefore, even now, in order to develop an oxidation-reduction polymer with excellent performance, the development of new materials beyond limitations of conventional materials is required.

Under this background, the present inventors have repeatedly studied polymers useful for immobilizing an electron transfer mediator and an enzyme for an electrochemical biosensor, and as a result, they have confirmed that when a polymer comprising pentafluorophenyl (PFP) ester is used, it has high reactivity with an amine functional group, less hydrolysis and generally good solubility in an organic solvent, and it has less steric hindrance and toxicity compared to polymers having a similar structure, and in particular, they have confirmed that it is useful for an implantable device in which a part of a sensor is inserted into a human body such as a continuous blood glucose monitoring sensor, thereby completing the present invention.

### [DISCLOSURE]

### [TECHNICAL PROBLEM]

An object of the present invention is to provide a polymer for manufacturing an oxidation-reduction polymer material comprising pentafluorophenyl ester.

Another object of the present invention is to provide an oxidation-reduction polymer material thin film and an electrochemical biosensor comprising a polymer for manufacturing the transition metal complex and the oxidation-reduction polymer material.

### [TECHNICAL SOLUTION]

As one aspect to solve the above objects, the present invention relates to a polymer for manufacturing an oxidation-reduction polymer material comprising pentafluorophenyl ester, an oxidation-reduction polymer for an electrochemical biosensor comprising the polymer and a transition metal complex, an oxidation-reduction polymer material thin film comprising an oxidation-reduction polymer for an electrochemical biosensor prepared therefrom, and an electrochemical biosensor, for example, a blood glucose sensor comprising thereof.

### [ADVANTAGEOUS EFFECTS]

The polypentafluorophenyl ester-based polymer according to the present invention has high reactivity with an amine functional group when used for an electrochemical sensor, less hydrolysis, high solubility in an organic solvent, and low steric hindrance and toxicity or side effects, and thus, in particular, it is useful for an implantable device in which a part of the sensor is placed inside a human body, such as a continuous blood glucose monitoring sensor.

### [BRIEF DESCRIPTION OF THE DRAWINGS]

FIG. 1 is a graph showing the reaction monitoring result through ¹⁹F NMR after adding the osmium complex **22** to the PFPA polymer **P1** according to the present invention.
FIG. 2 is a graph of confirming ¹⁹F NMR when the final reaction is progressed for the PFPA polymer **(36)** and osmium complex **(22, 33),** and the ratio of the oxidation-reduction polymer **(37, 38)** predicted through this.
FIG. 3 is the ¹⁹F NMR data confirming synthesis of an oxidation-reduction polymer comprising a transition metal complex comprising a C-N ligand and a PFP polymer of Example 1-4.
Fig. 4 is the ¹⁹F NMR data confirming synthesis of an oxidation-reduction polymer comprising a transition metal complex comprising a C-N ligand and a PFP polymer of Example 1-5.
FIG. 5 is a schematic diagram of the composition of the oxidation-reduction polymer and enzyme and the crosslinking method on the SPCEs electrode surface.
FIG. 6a is a graph showing the change in current when the glucose concentration is increased to 0.01, 0.05, 0.1, 0.5, 1, 2, 4, 8, 16, 32, 64, 100 mM per 200 seconds in the oxidation-reduction polymers (37 and 38) according to the present invention (top), change in current depending on the glucose concentration (bottom), the low concentration section (0 ~ 1 mM) in left and the total section (0 ~ 100 mM) in right.

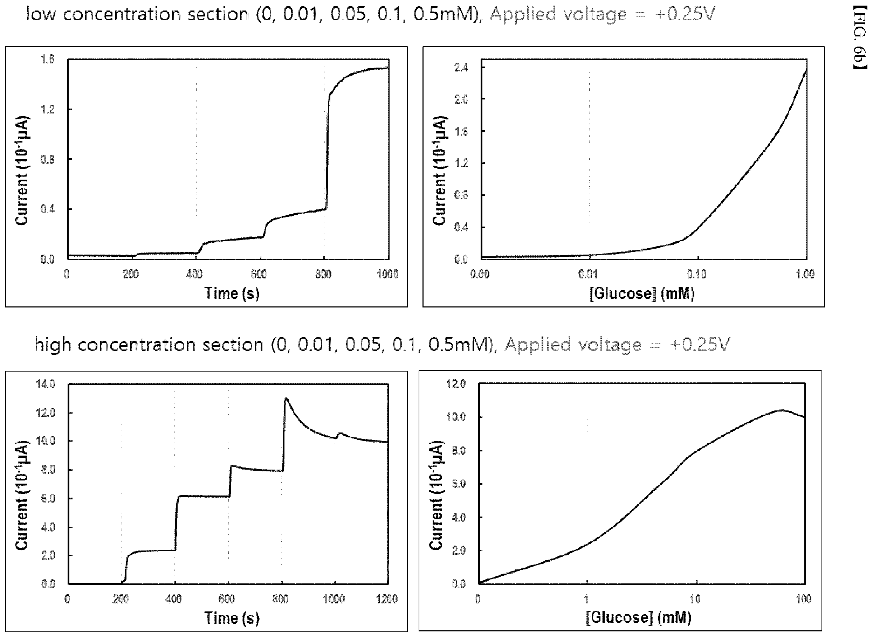

FIG. 6b is a graph showing the change in current when the glucose concentration is increased to 0.01, 0.05, 0.1, 0.5 mM per 200 seconds in the oxidation-reduction polymer (oxidation-reduction polymer 1 comprising a transition metal complex comprising a C-N ligand and a PFP polymer) according to the present invention (top-left), change in current depending on the glucose concentration (top-right) and the change in current when the glucose concentration is increased to 0, 1, 5, 10, 50, 100 mM per 200 seconds (bottom-left) and change in current according to the glucose concentration (bottom-right).

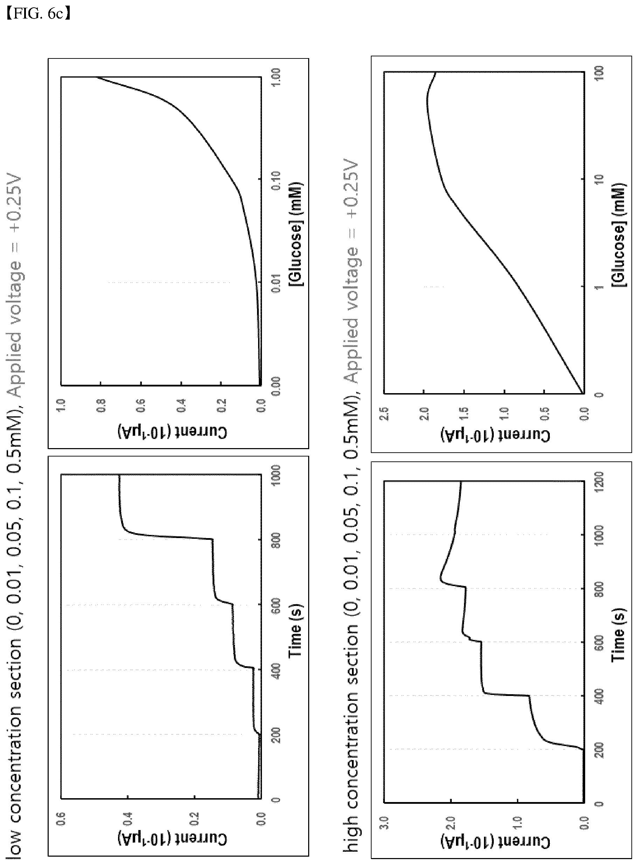

FIG. 6c is a graph showing the change in current when the glucose concentration is increased to 0.01, 0.05, 0.1, 0.5 mM per 200 seconds in the oxidation-reduction polymer (oxidation-reduction polymer 1 comprising a transition metal complex comprising a C-N ligand and a PFP polymer) according to the present invention (top-left), change in current depending on the glucose concentration (top-right) and the change in current when the glucose concentration is increased to 0, 1, 5, 10, 50, 100 mM per 200 seconds (bottom-left) and change in current according to the glucose concentration (bottom-right).
FIGs. 7a to 7d are graphs showing the result of measuring the change in current before and after the cyclic voltammetry test of the oxidation-reduction polymers according to the present invention (37, 38 and oxidation-reduction polymers 1 and 2 comprising a transition metal complex comprising a C-N ligand and a PFP polymer). In FIGs. 7a to 7d, CV represents a value measured immediately after completing an electrode, and after CV represents a value measured after stirring in a PBS solution for 1 hour.

### [BEST MODE]

Hereinafter, the present invention will be described in more detail.

The polymer for manufacturing an oxidation-reduction polymer material according to the present invention is based on polypentafluorophenyl ester, and specifically, is used as a polymer precursor of an oxidation-reduction polymer material, comprising a repeating unit derived from pentafluorophenyl ester. Preferably, the polymer may form an oxidation-reduction polymer material for an electron transfer mediator with a crosslinking material having a reaction group comprising an amine group and a transition metal complex.

As one example, the polymer for manufacturing an oxidation-reduction polymer material according to the present invention may be a polymer for manufacturing an oxidation-reduction polymer material, which is prepared by introducing primary and secondary amine-based compounds as a crosslinking material comprising an amine group into the polypentafluorophenyl ester through aminolysis.

A non-limitative example of the polymer for an oxidation-reduction polymer material may be one or more kinds of polymers selected from the group consisting of a polypentafluorophenyl acrylate (PPFPA) homopolymer, a polypentafluorophenyl methacrylate (PPFPM) homopolymer, a polypentafluorophenyl acrylate-polydimethylacrylamide (PPFPA-PDMA) copolymer, a polypentafluorophenyl methacrylate-polydimethylacrylamide (PPFPM-PDMA) copolymer, a polypentafluorophenyl acrylate-polyacrylamide (PPFPA-PAA) copolymer, a polypentafluorophenyl methacrylate-polyacrylamide (PPFPM-PAA) copolymer, but not limited thereto.

In one example, the polymer for manufacturing an oxidation-reduction polymer material according to the present invention may have a structure of Chemical formula 1 or 2 below:

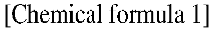

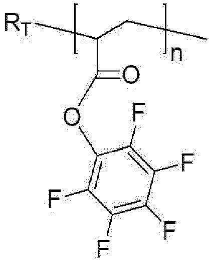

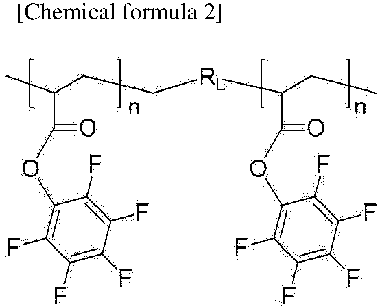

In the Chemical formula 1 or 2,
R_{T} and R_{L} are each independently selected from the group consisting of a substituted or non-substituted alkylene group having 1 to 20 carbon atoms, a substituted or non-substituted cycloalkylene group having 1 to 20 carbon atoms, a substituted or non-substituted ethylene glycol group having 3 to 30 carbon atoms, a substituted or non-substituted arylene group having 6 to 30 carbon atoms, a substituted or non-substituted heteroarylene group having 3 to 30 carbon atoms, a substituted or non-substituted alkenyl group having 2 to 40 carbon atoms and a substituted or non-substituted alkynyl group having 2 to 40 carbon atoms; and
the n is an integer of 10 to 300.

In the present invention, "substitution" may be that at least one hydrogen atom is 1 kind to 3 kinds selected from the group consisting of a halogen atom (for example, F, Cl, Br, or I), a cyano group, a hydroxyl group, a thiol group, a nitro group, an amino group, an imino group, an azido group, an amidino group, a hydrazine group, a hydrazono group, an oxo group, a carbonyl group, a carbamyl group, an ester group, an ether group, a carboxyl group or a salt thereof, a sulfonic acid group or a salt thereof, phosphate or a salt thereof, an alkyl group having 1-6 carbon atoms, a haloalkyl group having 1-6 carbon atoms, an alkenyl group having 2 to 6 carbon atoms, a haloalkenyl group having 2 to 6 carbon atoms, an alkynyl group having 2 to 6 carbon atoms, a haloalkynyl group having 2 to 6 carbon atoms, an alkoxy group having 1 to 6 carbon atoms, a haloalkoxy group having 1 to 6 carbon atoms, an alkylthio group having 1 to 6 carbon atoms, a heterocycloalkyl group having a carbocyclic ring of 1 to 9, an aryl group having 6 to 10 carbon atoms, an aryloxy group having 6 to 10 carbon atoms, an arylthiol group having 6 to 10 carbon atoms, a heteroalkyl group having a carbocyclic ring of 1 to 9, a heteroaryloxy group having a carbocyclic ring of 1 to 9 and a heteroarylthio group having a carbocyclic ring having 1 to 9 carbon atoms, unless otherwise mentioned.

Preferably, the R_{T} and R_{L} may be each independently selected from the group consisting of polydimethylacrylamide (PDMA), polyacrylamide (PAA), polystyrene (PS), polyethylene glycol (PEG), polyethyleneoxide (PEO), polymethylmethacrylate (PMMA), polyvinylimidazole (PVI), polyvinylpyridine (PVP) and polysiloxane (PDMS) with a molecular weight of 1,000 g/mol ~ 50,000 g/mol.

As one example, the polymer for manufacturing an oxidation-reduction polymer material according to the present invention may have a weight average molecular weight within a range of 1,000 g/mol to 500,000 g/mol, but not limited thereto.

As another aspect, the present invention relates to an oxidation-reduction polymer material for an electrochemical sensor in which a transition metal complex is introduced to the polypentafluorophenyl ester-based polymer.

For example, this oxidation-reduction polymer material may be prepared by functionalizing the polypentafluorophenyl ester-based polymer by introducing a compound comprising a functional group selected from the group consisting of an amine group, an ammonium group, a halogen group, an epoxy group, an azide group, an acrylate group, an alkenyl group, an alkynyl group, a thiol group, an isocyanate, an alcohol group and a silane group, and binding a transition metal complex to the polymer functionalized as such.

In addition, the oxidation-reduction polymer material may be prepared by functionalizing the polypentafluorophenyl ester-based polymer by introducing a functional group such as the crosslinking material such as an amine group, an ammonium group, a thiol group, an alcohol group and the like through a substitution reaction and an addition reaction to a ligand of a transition metal complex, and binding the transition metal complex functionalized as such and the polymer according to the present invention, instead of functionalizing the polymer.

Otherwise, the polymer material may be prepared by functionalizing all the polypentafluorophenyl ester-based polymer and a transition metal complex using a crosslinking material comprising a functional group selected from the group consisting of an amine group, an ammonium group, a halogen group, an epoxy group, an azide group, an acrylate group, an alkenyl group, an alkynyl group, a thiol group, an isocyanate, an alcohol group and a silane group, and binding them each other.

Preferably, in the oxidation-reduction polymer material according to the present invention, a functional group selected from the group consisting of primary and secondary amine groups and ammonium groups and a transition metal complex are combined to the polypentafluorophenyl ester-based polymer.

Specifically, the transition metal complex may have a structure of Chemical formula 3 or 4 below.

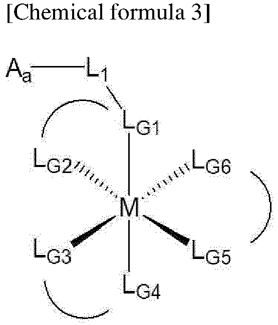

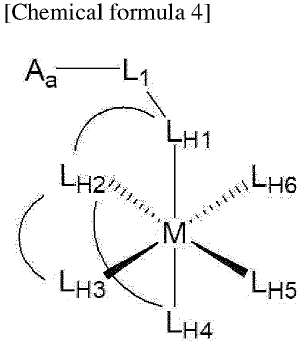

In the Chemical formula 3 or 4,
M is one kind of transition metals selected from the group consisting of Os, Rh, Ru, Ir, Fe and Co; and
in the Chemical formula 3, L_{G1} and L_{G2} are combined with each other to form a bidentate ligand selected from Chemical formula 5 to 6 below; and
L_{G3} and L_{G4} are combined with each other to form a bidentate ligand selected from Chemical formula 5 to 6 below; and L_{G5} and L_{G6} are combined with each other to form a bidentate ligand selected from Chemical formula 5 to 6 below; and

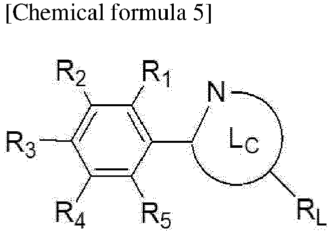

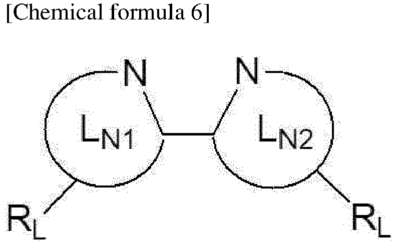

in the Chemical formula 5, Lc is a heterocyclic compound comprising one or more nitrogen atoms, and is linked with the chemical formula of benzene at position 2; and
in the Chemical formula 6, L_{N} is a heterocyclic compound comprising one or more nitrogen atoms, and L_{N1} and L_{N2} are linked to each other at position 2, respectively.
R_{L} is all functional groups of heterocyclic compounds Lc, L_{N1} and L_{N2}.

In one aspect, the R₁, R₂, R₃, R₄, R₅ and R_{L} may be each independently selected from the group consisting of a substituted or non-substituted alkyl group having 1 to 10 carbon atoms, a substituted or non-substituted ethylene glycol group having 2 to 20 carbon atoms, a substituted or non-substituted alcohol group having 1 to 20 carbon atoms, a substituted or non-substituted alkyl halogen group having 1 to 20 carbon atoms, a substituted or non-substituted thiol group having 1 to 20 carbon atoms, a substituted or non-substituted alkyl azide group having 3 to 20 carbon atoms, a substituted or non-substituted aryl azide group having 7 to 30 carbon atoms, a substituted or non-substituted alkenyl group having 2 to 40 carbon atoms, a substituted or non-substituted alkynyl group having 2 to 40 carbon atoms, a cyano group, a halogen group, heavy hydrogen and hydrogen.

In one aspect, the L_{H1}, L_{H3} and L_{H4} of the Chemical formula 4 may be combined with each other centering on L_{H2} to form a tetradentate ligand represented by Chemical formula 7 below.

Then, the L_{H5} and L_{H6} may form a monodentate ligand for transition metal M as represented in Chemical formula 8, respectively; or L_{H5} and L_{H6} may be combined with each other to form a bidentate ligand for transition metal M as represented by Chemical formula 9 below, respectively; and

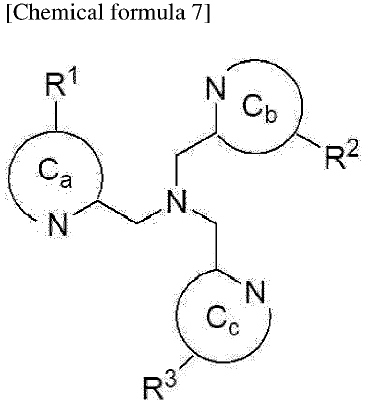

In the Chemical formula 7,
Cₐ, C_{b} and C_{c} are heterocyclic compounds comprising one or more nitrogen atoms, and preferably, the heterocyclic compound may be linked to methylamine at position 2 thereof, and 3 nitrogens of the three hetero rings, and 1 nitrogen at the center connecting the three hetero rings may be linked to transition metal M.
R₁, R₂, R₃ means all functional groups of the heterocyclic compounds Cₐ, C_{b}, C_{c}.

In one aspect, the R₁, R₂ and R₃ may be each independently selected from the group consisting of a substituted or non-substituted alkyl group having 1 to 10 carbon atoms, a substituted or non-substituted ethylene glycol groups having 2 to 20 carbon atoms, a substituted or non-substituted alcohol group having 1 to 20 carbon atoms, a substituted or non-substituted alkyl halogen group having 1 to 20 carbon atoms, a substituted or non-substituted thiol group having 1 to 20 carbon atoms, a substituted or non-substituted alkyl azide group having 3 to 20 carbon atoms, a substituted or non-substituted aryl azide group having 7 to 30 carbon atoms, a substituted or non-substituted alkenyl group having 2 to 40 carbon atoms, a substituted or non-substituted alkylnyl group having 2 to 40 carbon atoms, a cyano group, a halogen group, heavy hydrogen and hydrogen. Preferably, a reaction group capable of linking to the polymer may be each independently introduced.

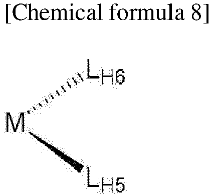

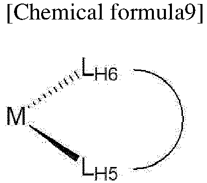

In the Chemical formula 8,
L_{H5} and L_{H6} may be independently a monodentate ligand, and are each independently a heterocyclic compound comprising one or more -H, -F, -Cl, -Br, -I, -NO₂, -NCCH₃, -CO, -OH₂, - NH₃ or nitrogen atoms.

In the Chemical formula 9,
L_{H5}-L_{H6} may be bidentate ligands, and are catechol, acetylacetone, 2-picolinic acid, 2-pyridinecarboxamide, 2,2-bipyridine, or 2,2-bithiazole.

The Li may be independently selected from the group consisting of a substituted or non-substituted alkylene group having 1 to 20 carbon atoms, a substituted or non-substituted cycloalkylene group having 1 to 20 carbon atoms, a substituted or non-substituted ethylene glycol group having 2 to 30 carbon atoms, a substituted or non-substituted arylene group having 6 to 30 carbon atoms, and a substituted or non-substituted heteroarylene group having 3 to 30 carbon atoms; and
the Aₐ is selected from the group consisting of an amine group and ammonium group.

One aspect of the present invention relates to a method for preparation of an oxidation-reduction polymer material thin film, comprising coating an oxidation-reduction polymer material comprising the polymer to an electrode, and then hardening the coated electrode, and an oxidation-reduction polymer material thin film prepared thereby, and an electrochemical biosensor comprising the same.

In addition, one additional aspect of the present invention relates to a sensing layer for an electrochemical biosensor comprising an C enzyme capable of redoxing a liquid biological sample; and an electron transfer mediator comprising the transition metal complex.

Oxidoreductase refers to an enzyme that catalyzes a redox reaction in a living body, and in the present invention, in case of a target material to be measured, for example, a biosensor, it refers to an enzyme which is reduced by reacting with the target material to be measured. The enzyme reduced as such reacts with an electron transfer mediator, and then, the target material is quantified by measuring a signal such as a change in current generated. The oxidoreductase available in the present invention may be one or more kinds selected from the group consisting of various kinds of dehydrogenase, oxidase, esterase and the like, and may be used by selecting an enzyme using the target material as a substrate among enzymes belonging to the enzyme group, depending on the redox or detection target material.

More specifically, the oxidoreductase may be one or more kinds selected from the group consisting of glucose dehydrogenase, glutamate dehydrogenase, glucose oxidase, cholesterol oxidase, cholesterol esterase, lactate oxidase, ascorbic acid oxidase, alcohol oxidase, alcohol dehydrogenase, bilirubin oxidase, and the like.

On the other hand, the oxidoreductase may comprise a cofactor which plays a role of storing hydrogen stolen by the oxidoreductase from the target material to be measured (for example, target material) together, and for example, it may be one or more kinds selected from the group consisting of flavin adenine dinucleotide (FAD), nicotinamide adenine dinucleotide (NAD), pyrroloquinoline quinone (PQQ), and the like.

For example, when the blood glucose concentration is to be measured, glucose dehydrogenase (GDH) may be used as the oxidoreductase, and the glucose dehydrogenase may be flavin adenine dinucleotide-glucose dehydrogenase (FAD-GDH) comprising FAD as a cofactor, and/or nicotinamide adenine dinucleotide- glucose dehydrogenase comprising FAD-GDH as a cofactor.

In a specific embodiment, the usable oxidoreductase may be one or more kinds selected from the group consisting of FAD-GDH (for example, EC 1.1.99.10, etc.), NAD-GDH (for example, EC 1.1.1.47, etc.), PQQ-GDH (for example, EC1.1.5.2, etc.), glutamate dehydrogenase (for example, EC 1.4.1.2, etc.), glucose oxidase (for example, EC 1.1.3.4, etc.), cholesterol oxidase (for example, EC 1.1.3.6, etc.), cholesterol esterase (for example, EC 3.1.1.13, etc.), lactate oxidase (for example, EC 1.1.3.2, etc.), ascorbate oxidase (for example, EC 1.10.3.3, etc.), alcohol oxidase (for example, EC 1.1.3.13, etc.), alcohol dehydrogenase (for example, EC 1.1.1.1, etc.), bilirubin oxidase (for example, EC 1.3.3.5, etc.), and the like.

Most preferably, the oxidoreductase is glucose dehydrogenase capable of maintaining the activity of 70% or more in a 37°C buffer solution for 1 week.

The sensing layer according to the present invention may contain a redox polymer of 20 to 700 parts by weight, for example, 60 to 700 parts by weight, or 30 to 340 parts by weight, based on oxidoreductase 100 parts by weight. The content of the redox polymer may be appropriately adjusted according to the activity of oxidoreductase.

Moreover, the sensing layer according to the present invention may further comprise a carbon nanotube for increasing the membrane performance. Specifically, the carbon nanotube may further increase the performance of the sensing layer as the electron transfer rate is increased when a transition metal complex, particularly, osmium is used together.

In addition, the sensing layer according to the present invention may further comprise a crosslinking agent.

On the other hand, the sensing layer according to the present invention may further comprise one or more kinds of additives selected from the group consisting of a surfactant, an aqueous polymer, a tertiary ammonium salt, a fatty acid, a thickener, and the like, for a role of a role of a dispersing agent during reagent dissolution, an adhesive during reagent preparation, a stabilizer for storage for a long period of time, and the like.

The surfactant may play a role of distributing the composition evenly on an electrode to be aliquoted in a uniform thickness, when the composition is aliquoted. As the surfactant, one or more kinds selected from the group consisting of Triton X-100, sodium dodecyl sulfate, perfluorooctane sulfonate, sodium stearate, and the like. The reagent composition according to the present invention may contain the surfactant in an amount of 3 to 5 parts by weight, for example, 10 to 25 parts by weight, based on oxidoreductase 100 parts by weight, so as to appropriately perform the role of evenly distributing the reagent on an electrode to be aliquoted with a uniform thickness when the reagent is aliquoted. For example, when oxidoreductase having an activity of 700 U/mg is used, 10 to 25 parts by weight of a surfactant based on oxidoreductase 100 parts by weight may be contained, and when the activity of oxidoreductase is higher than this, the content of the surfactant may be adjusted lower than this.

The aqueous polymer is a polymer supporter of a reagent composition, which performs a role of helping stabilization and dispersing of an enzyme. As the aqueous polymer, one or more kinds selected from the group consisting of polyvinyl pyrrolidone (PVP), polyvinyl alcohol (PVA), polyperfluoro sulfonate, hydroxyethyl cellulose (HEC), hydroxypropyl cellulose (HPC), carboxy methyl cellulose (CMC), cellulose acetate, polyamide, and the like may be used. The reagent composition according to the present invention, may contain the aqueous polymer of 10 to 70 parts by weight, for example, 30 to 70 parts by weight, based on oxidoreductase 100 parts by weight, in order to sufficiently and appropriately exhibiting a role of helping stabilization and dispersing of oxidoreductase. For example, when oxidoreductase having an activity of 700 U/mg is used, the aqueous polymer of 30 to 70 parts by weight based on oxidoreductase 100 parts by weight may be contained, and when the activity of oxidoreductase is higher than this, the content of the aqueous polymer may be adjusted lower than this.

The aqueous polymer may have a weight average molecular weight of about 2,500 g/mol to 3,000,000 g/mol, for example, 5,000 g/mol to 1,000,000 g/mol, so as to effectively perform a role of helping stabilization and dispersing of the supporter and enzyme.

The thickener plays a role of strongly attaching a reagent to an electrode. As the thickener, one or more kinds selected from the group consisting of natrosol, diethylaminoethyl-dextran hydrochloride (DEAE-Dextran hydrochloride) and the like may be used. The electrochemical sensor according to the present invention, may contain the thickener in an amount of 10 to 90 parts by weight, for example, 30 to 90 parts by weight, based on oxidoreductase 100 parts by weight, in order to strongly attach the redox polymer according to the present invention to an electrode. For example, when oxidoreductase having an activity of 700 U/mg is used, the thickener of 30 to 90 parts by weight based on oxidoreductase 100 parts by weight may be contained, and when the activity of oxidoreductase is higher than this, the content of the thickener may be adjusted lower than this.

As other aspect, the present invention may be a device, preferably, an insertable device, for example, a device insertable into a human body, comprising this organic electron transfer mediator. In addition, preferably, the device may be an electrochemical biosensor, more preferably, an electrochemical glucose (blood glucose) sensor.

Specifically, there is no limitation on the type of the electrochemical biosensor, but it may be a continuous blood glucose monitoring sensor.

As the composition of this continuous blood glucose monitoring sensor, the present invention, may comprise for example, an electrode, an insulator, a substrate, a sensing layer comprising the redox polymer and oxidoreductase, a diffusion layer, a protection layer, and the like. In case of the electrode, two kinds of electrodes such as a working electrode and a counter electrode, and may comprise three kinds of electrodes such as a working electrode, a counter electrode and a reference electrode. In one embodiment, the biosensor according to the present invention may be an electrochemical biosensor produced by applying a reagent composition comprising a redox polymer comprising the organic series electron transfer mediator of Chemical formula 1 and an enzyme capable of redoxing a liquid biological sample, to a substrate having at least two, preferably, two or three electrodes, and drying. For example, in the electrochemical biosensor, a planar electrochemical biosensor, in which a working electrode and a counter electrode are equipped on opposite sides each other, and a sensing layer comprising a redox polymer having the organic series electron transfer mediator according to the present invention is laminated on the working electrode, and an insulator, a diffusion layer and a protective layer are laminated in order on both sides of the substrate equipped with the working electrode and counter electrode, is provided.

As a specific aspect, the substrate may be made of one or more kinds selected from the group consisting of PET (polyethylene terephthalate), PC (polycarbonate) and PI (polyimide).

In addition, the working electrode may use a carbon, gold, platinum, silver or silver/silver chloride electrode.

Furthermore, in case of an electrochemical biosensor having 2 electrodes, a counter electrode also plays a role of a reference electrode, and therefore, as the counter electrode, a gold, platinum, silver or silver/silver chloride electrode may be used, and in case of an electrochemical biosensor of three electrodes comprising a reference electrode, as the reference electrode, a gold, platinum, silver or silver/silver chloride electrode may be used, and as the counter electrode, a carbon electrode may be used.

As the diffusion layer, Nafion, cellulose acetate and silicone rubber may be used, and as the protective layer, silicone rubber, polyurethane, polyurethane-based copolymer and the liker may be used, but not limited thereto.

As a non-limited example, in case of two electrodes, the counter electrode also plays a role of the reference electrode, and therefore, silver chloride or silver may be used, and in case of three electrodes, as the reference electrode, silver chloride or silver may be used, and as the counter electrode, a carbon electrode may be used.

A specific embodiment of the present invention illustrates a biosensor for measuring glucose as an applicable example of an electrochemical biosensor, but by changing the type of enzyme comprised in the reagent composition of the present invention, it may be applied for a biosensor for quantification of various substances such as cholesterol, lactate, creatinine, hydrogen peroxide, alcohol, amino acid, and glutamate.

### [MODE FOR INVENTION]

Hereinafter, the present invention will be described in more detail by the following examples. However, the following examples illustrate the present invention only, but the content of the present invention is not limited by the following examples.

### [Example]

### Experimental materials

### Reference example 1. Reagents and samples

Reagents commercially purchased from Aldrich company and Acros company, TCI company and Alfa aesar company were used without particular purification, and ethylene diamine, pyridine, 1-methylimidazole and triethylamine were used by filling basic alumina in a 10 mL pipette and then filtering. Several solvents purchased from Daejung and Samchun as follows were purified and used. Anhydrous dichloromethane and anhydrous N,N-dimethylformamide were obtained by adding calcium hydride and distilling, and anhydrous acetonitrile was obtained by distilling using P₂O₅. Diethyl ether was distilled using sodium and benzophenone. For thin-layer chromatography for analysis, Merck silica gel 60 F₂₅₄ glass plate was used, and fluorescence was observed using a dual short-wavelength (254 nm)/long-wavelength (365 nm) UV lamp. For column chromatography, Aldrich basic aluminum oxide or Alfa neutral aluminum oxide was used. As screen printed carbon electrodes (SPCEs), SE102 product of Zensor company was used.

### Reference example 2. Equipment

Hydrogen nuclear magnetic resonance (¹H NMR), carbon nuclear magnetic resonance (¹³C NMR) and fluorine nuclear magnetic resonance (¹⁹F NMR) spectra were obtained using Varian Inova 400 (400 MHz for ¹H, and 100 MHz for ¹³C) spectrometer. All chemical shifts were expressed in a ppm unit on the basis of tetramethylsilane (δ 0.00) or deuterated chloroform (δ 7.26 for CDCl₃ in ¹H NMR, δ 77.2 for CDCl₃ in ¹³C NMR), deuterated dimethylsulfoxide (δ 2.50 for DMSO in ¹H NMR, δ 39.52 for DMSO in ¹³C NMR), and deuterated acetonitrile (δ 1.94 for CD₃CN in ¹H NMR). In case of fluorine nuclear magnetic resonance (¹⁹F NMR), trifluorotoluene (CF₃-Ar, δ -69.27 for CD₃OD in ¹⁹F NMR) was added in a small quantity to deuterated methanol and based on this, it was expressed in a ppm unit. Measurement of cyclic voltammograms (CV) were performed by a model of CHI1040C of CH Instruments company. It was measured using a well-washed carbon glass electrode with a diameter of 3mm as a working electrode, an Ag/AgCl electrode as a reference electrode, and a Pt electrode as a counter electrode, at a scanning rate of 10 ~ 50 mV/s. A mass spectrum was obtained by ESI-Iontrap in case of low resolution and ESI-orbitrap in case of high resolution, of LTQ XL model of ThermoFisher Scientific company in the organic chemistry research center of Sogang University. Single crystal X-ray diffraction analysis was commissioned by Korea Research Institute of Chemical Technology and the crystal structure could be identified using Bruker SMART APEX II model.

### Example 1. Oxidation-reduction polymer synthesis using PFPA polymer matrix

An object of this experiment was to synthesize an oxidation-reduction polymer through a reaction with an electron transfer mediator comprising an amine group (-NH₂) at the terminus from a pentafluorophenylacrylate (PFPA)-based polymer matrix. At first, using AIBN, a DMA : PFPA polymer (**P1**) was synthesized through radical polymerization of pentafluorophenylacrylate (PFPA) and dimethylacrylamide (DMA) at a ratio of 5:5.

### 1-1. Synthesis of Poly(N,N-dimethyl acrylamide-co-pentafluorophenyl acrylate) (P1)

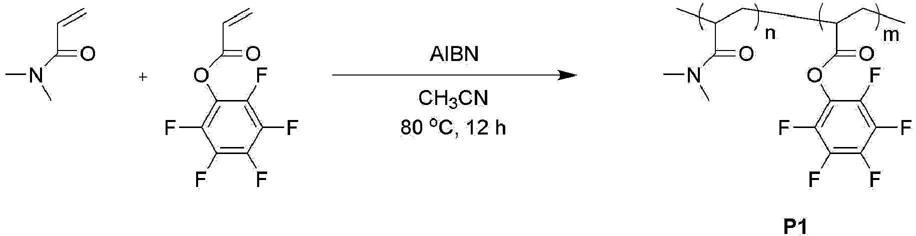

### [Synthesis of DMA : PFPA polymer]

N,N-dimethyl acrylamide (150 mg, 1.51 mmol), pentafluorophenyl acrylate (360 mg, 1.51 mmol), azobisisobutyronitrile (AIBN, 7 mg, 0.043 mmol) are added to a glass culturing tube and acetonitrile (8 mL) is added to make a mixed solution. After that, argon gas is blown for 10 minutes to create an argon atmosphere in the glass culturing tube and then it is refluxed at 80 °C for 12 hours. After completing the reaction, the solvent is removed using a rotary evaporator, and CH₂Cl₂ is added to make a mixed solution. A polymer produced by repeating slowly dropping the mixed solution to n-hexane to obtain a precipitate three times is purified. By drying a polymer obtained by filtering the precipitate and washing with n-hexane in a vacuum for 6 hours or more, product **P1** in a white solid form was obtained. (482 mg, 95%) ¹H NMR (400 MHz, CDCl₃) *δ* 2.81 to 3.34 ppm (br, 7H, -CH- in the backbone of PPFPA and -N(CH₃)₂ from PDMA), 2.47 to 2.65 ppm (br, 1H, -CH- in the backbone of PDMA), 1.13 to 2.22 ppm (br, 4H, -CH₂- in the backbone of PDMA and PPFPA), ¹⁹F NMR (400 MHz, CDCl₃) *δ* -152.90 (br, ortho), -157.21 (br, para), -162.15 (br, meta)

The synthesized polymer exhibited good solubility in methanol, acetonitrile, acetone and dichloromethane, but was not dissolved at all in water and diethyl ether.

### 1-2. Confirmation of whether PFPA polymer can function as a polymer matrix capable of immobilizing an electron transfer mediator and GDH

In order to confirm whether the reaction between the polymer **P1** synthesized in Example 1-1 and osmium complex **22** comprising an amine group at the terminus proceeded, it was observed through ¹⁹F NMR.

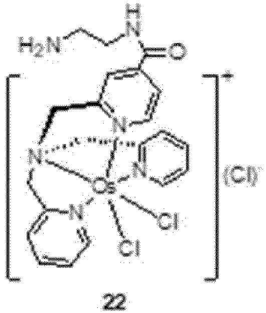

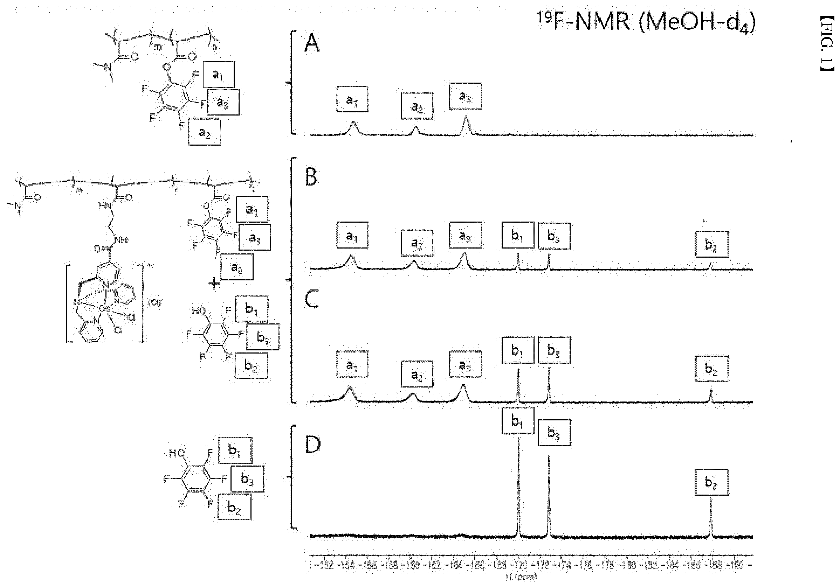

Complex No. **22** reacted in a form in which a zwitterion is Cl⁻, and NMR peaks were confirmed under a deuterated methanol solvent capable of dissolving both the complex and polymer. In addition, as it could not react with PFP ester when the amine group of complex No. **22** was present in an ammonium (-NH₄⁺) form, triethylamine (TEA) was added as much as the equivalent of the complex. The result was shown in FIG. 1. As could be confirmed in FIG. 1, initially, only polymer **P1** was confirmed by ¹⁹F NMR, and as a result, three broad peaks corresponding to PFPA were shown. Then, as soon as complex No. **22** was added, it was observed that three peaks of pentafluorophenol (PFPOH) in a sharp point form were additionally shown. These are peaks of PFPOH, a by-product produced by reaction of amine at the terminus of complex No. **22.** As time passed, a change in that the peaks of PFPOH increased and the peaks of PFPA decreased gradually, and after about 36 hours, all the peaks of PFPA disappeared and only the peaks of PFPOH remained. Through this result, it was determined that PFP ester could react with an amine group comprising a TPMA-based osmium complex, and the PFPA polymer could function as a polymer matrix capable of immobilizing an electron transfer mediator and GDH.

### 1-3. Synthesis of oxidation-reduction polymers (Redox polymers (36) and (37))

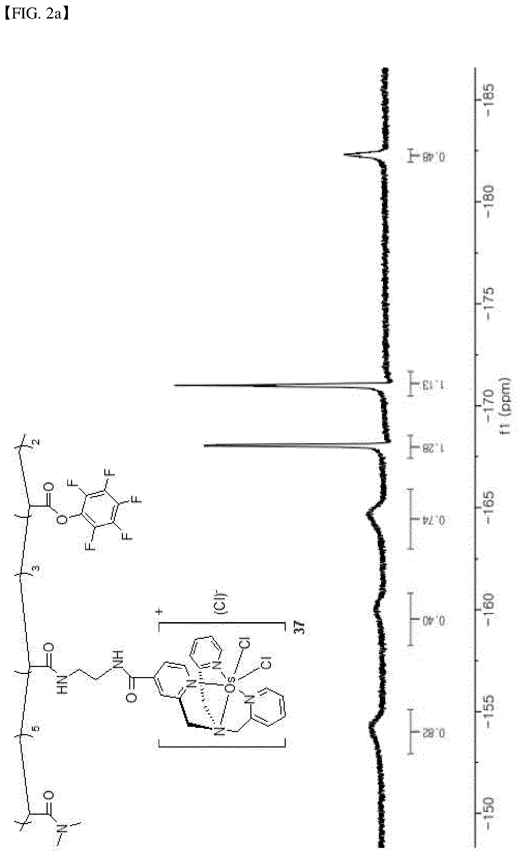

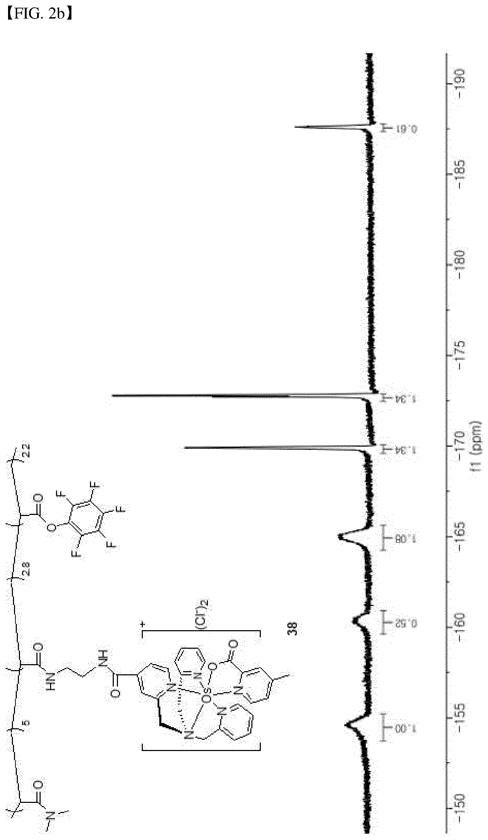

By reacting TPMA-based osmium complexes **22** and **33** comprising an amine functional group with the PFPA polymer **36,** oxidation-reduction polymers were synthesized (FIG. 2). In order to obtain oxidation-reduction polymers that could comprise an osmium complex by about 25-30% in an entire polymer chain, the reaction was progressed by calculating an equivalent ratio.

### 1) Synthesis of oxidation-reduction polymer (36)

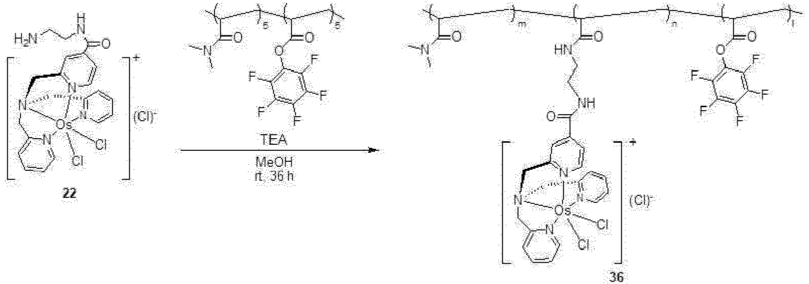

Starting material **22** (6 mg, 0.009 mmol) is dissolved in deuterated methanol (0.5 mL) in an NMR tube. The dissolved solution is added to starting material **P1** (5 mg) dissolved in deuterated methanol (0.5 mL) and then triethylamine (1.24 µL, 0.009 mmol) is added. After confirming the progress of the reaction through ¹⁹F NMR, a polymer produced by repeating slowly dropping the reaction solution into diethyl ether to obtain a precipitate three times is purified. The produced precipitate was filtered and washed with diethyl ether, and then dried in a vacuum for 4 hours or more to obtain product **36** in a brown solid form. (6 mg, 64%) ¹⁹F NMR (400 MHz, CD₃OD) *δ* -153.38 (br, ortho), -159.74 (br, para), -163.53 (br, meta)

### 2) Synthesis of oxidation-reduction polymer (37)

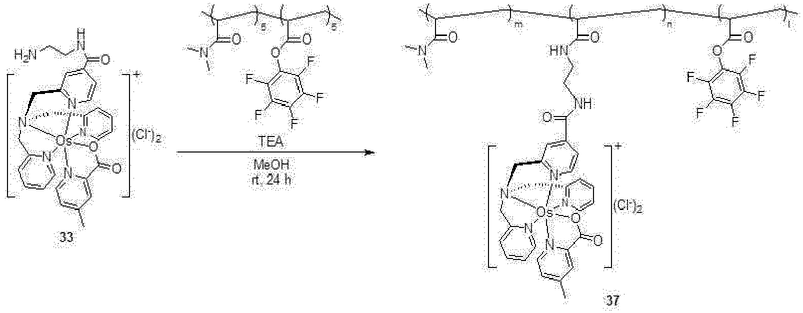

Starting material **33** (6.5 mg, 0.009 mmol) is dissolved in deuterated methanol (0.5 mL) in an NMR tube. The dissolved solution is added to starting material **P1** (5 mg) dissolved in deuterated methanol (0.5 mL) and then triethylamine (1.22 µL, 0.009 mmol) is added. After confirming the progress of the reaction through ¹⁹F NMR, a polymer produced by repeating slowly dropping the reaction solution into diethyl ether to obtain a precipitate three times is purified. The produced precipitate was filtered and washed with diethyl ether, and then dried in a vacuum for 4 hours or more to obtain product **37** in a brown solid form. (7.2 mg, 73%) ¹⁹F NMR (400 MHz, CD₃OD) *δ* -154.57 (br, ortho), -160.27 (br, para), -164.95 (br, meta)

The reaction between the added complex and PFPA polymer was confirmed through ¹⁹F NMR until there was no further change in the peak, and the ratio of the oxidation-reduction polymers was determined. It took about 24 ~ 36 hours for all the added osmium complexes to react. As the result of confirming the ratio of the two oxidation-reduction polymers through ¹⁹F NMR, it could be indirectly determined that in case of oxidation-reduction polymer **37,** the PFPA : PFPOH ratio was shown as 2 : 3, and the osmium complex occupied 30% of the total chain, and in case of **38,** the PFPA : PFPOH ratio was shown as 2.2 : 2.8, and 28% of the osmium complex was comprised in the polymer matrix. Both polymers were obtained by precipitation in diethyl ether, and unlike the starting material **36** polymer, all of the oxidation-reduction polymers were soluble in water. In addition, as confirmed below, when CV was measured, an oxidation-reduction potential similar to an osmium complex monomer was exhibited. Through this result, it was determined that an oxidation-reduction polymer comprising a TPMA-based osmium complex was successfully synthesized.

### 1-4. Synthesis of oxidation-reduction polymer comprising transition metal complex comprising C-N ligand and PFP polymer 1

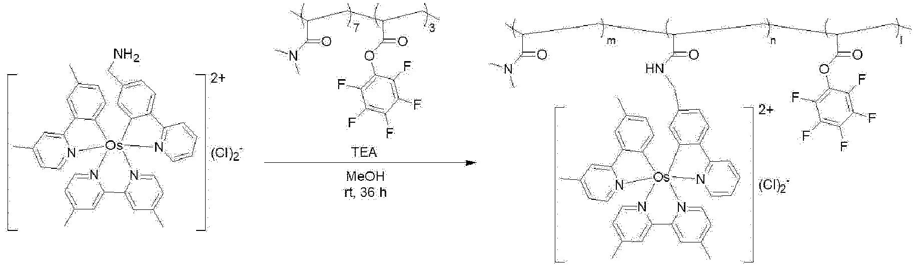

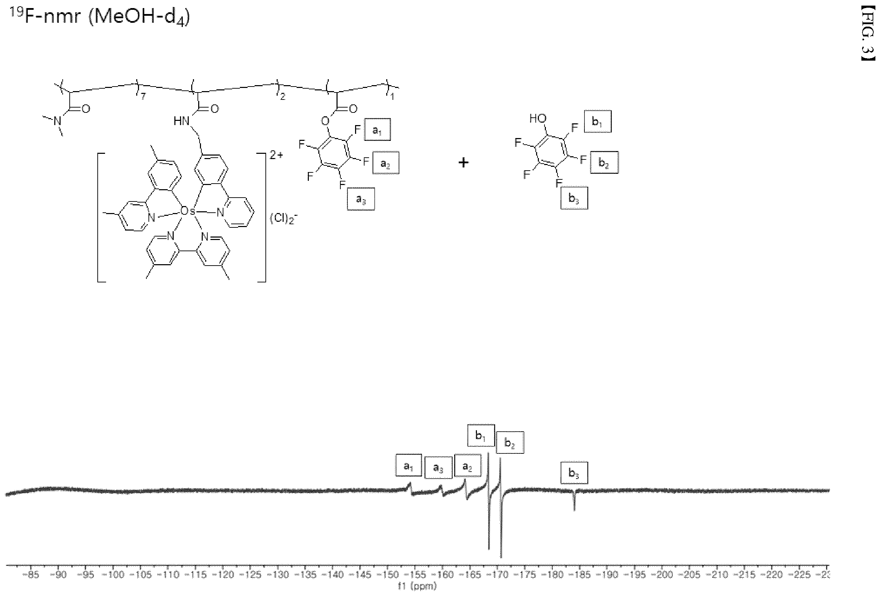

As a starting material, an osmium complex comprising a C-N ligand, [Os(2-(2-pyridinyl-κN)-5-methaneamine-phenyl-κC)(4,4'-dimethyl-2,2'-bipyridine)₂]Cl₂ (11 mg, 0.014 mmol) is dissolved in deuterated methanol (0.5 mL) in an NMR tube. The dissolved solution was added to a starting material PFP polymer (7:3) (17 mg) dissolved in deuterated methanol (0.5 mL), and then triethylamine (3.8 µL, 0.028 mmol) was added. After confirming the progress of the reaction through ¹⁹F NMR (FIG. 3), a polymer produced by repeating slowly dropping the reaction solution into diethyl ether to obtain a precipitate three times was purified. The produced precipitate was filtered and washed with diethyl ether, and then dried in a vacuum for 4 hours or more to obtain a product in a brown solid form.

### 1-5. Synthesis of oxidation-reduction polymer comprising transition metal complex comprising C-N ligand 2

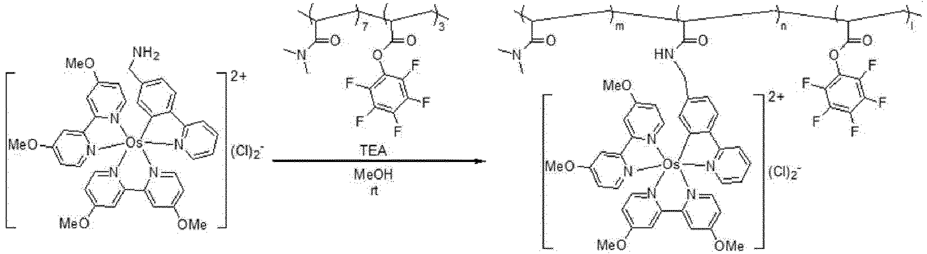

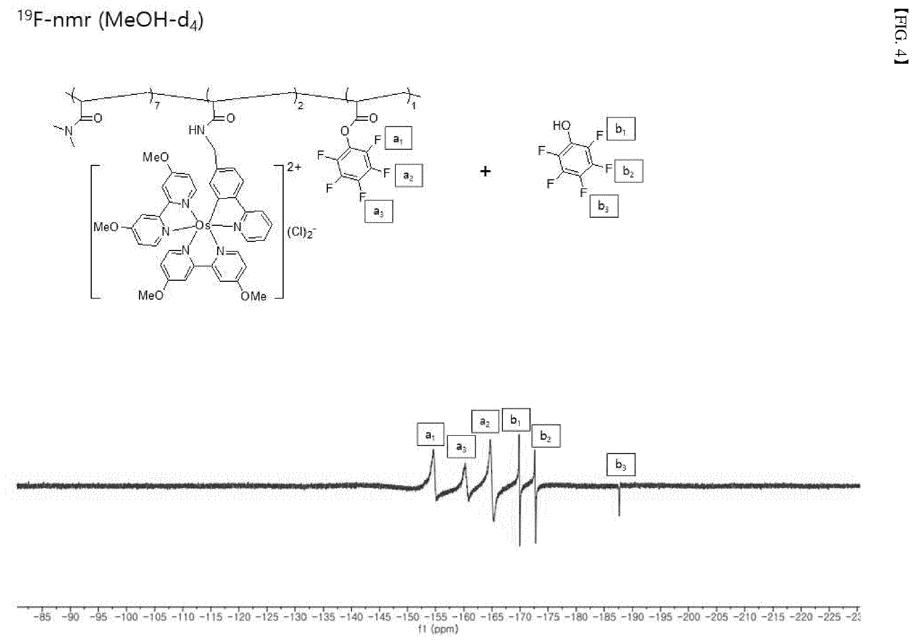

A starting material, [Os(2-(2-pyridinyl-κN)-5-methaneamine-phenyl-κC)(4,4'-dimethoxy-2,2'-bipyridine)₂]Cl₂ (9.7 mg, 0.012 mmol) is dissolved in deuterated methanol (0.5 mL) in an NMR tube. The dissolved solution was added to a starting material PFP polymer (7:3) (15 mg) dissolved in deuterated methanol (0.5 mL), and then triethylamine (3.1 µL, 0.024 mmol) was added. After confirming the progress of the reaction through ¹⁹F NMR (FIG. 4), a polymer produced by repeating slowly dropping the reaction solution into diethyl ether to obtain a precipitate three times was purified. The produced precipitate was filtered and washed with diethyl ether, and then dried in a vacuum for 4 hours or more to obtain a product in a brown solid form.

### Example 2. Immobilization of oxidation-reduction polymer and enzyme and test for response for glucose

Solution in which an oxidation-reduction polymer comprising an osmium complex and glucose dehydrogenase (GDH) were mixed was immobilized on an SPCEs electrode, and to confirm the response depending on the glucose concentration, the following test was performed.

### 2-1. Preparation of electrode in which oxidation-reduction polymer and glucose dehydrogenase are immobilized

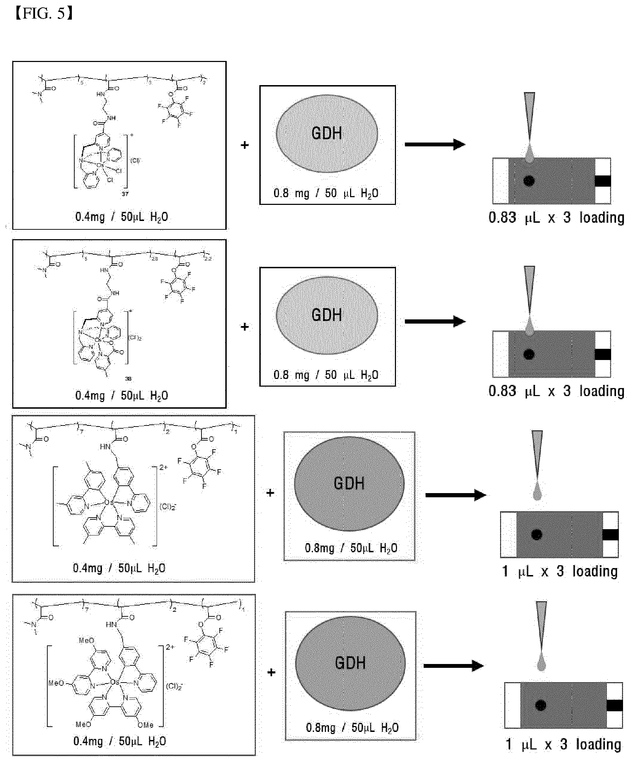

A mixed solution was prepared by mixing a solution in which an oxidation-reduction polymer 0.4 mg was dissolved in distilled water 50 µL and a solution in which glucose dehydrogenase (GDH) 0.8 mg was dissolved in distilled water 50 µL (v/v 1:1). This mixed solution of 0.83 µL in case of the oxidation-reduction polymers 37 and 38 and 1 µL in case of the oxidation-reduction polymers 1 and 2 comprising a transition metal complex comprising a C-N ligand was evenly placed on the SPCEs, and dried slowly at a room temperature. After completing drying, the mixed solution of 0.83 µL in case of the oxidation-reduction polymers 37 and 38 and 1 µL in case of the oxidation-reduction polymers 1 and 2 comprising a transition metal complex comprising a C-N ligand was placed again, and this process was repeated twice more. The electrode on which the mixed solution of 2.5 µL (polymers 37 and 38) and 3 µL (oxidation-reduction polymers 1 and 2 comprising a transition metal complex comprising a C-N ligand) in total was dried at a room temperature for 12 hours or more to prepare an electrode. The composition of the mediator and enzyme and the crosslinking method on the SPCEs electrode surface were schematically shown in FIG. 5.

### 2-2. Current measurement according to glucose concentration

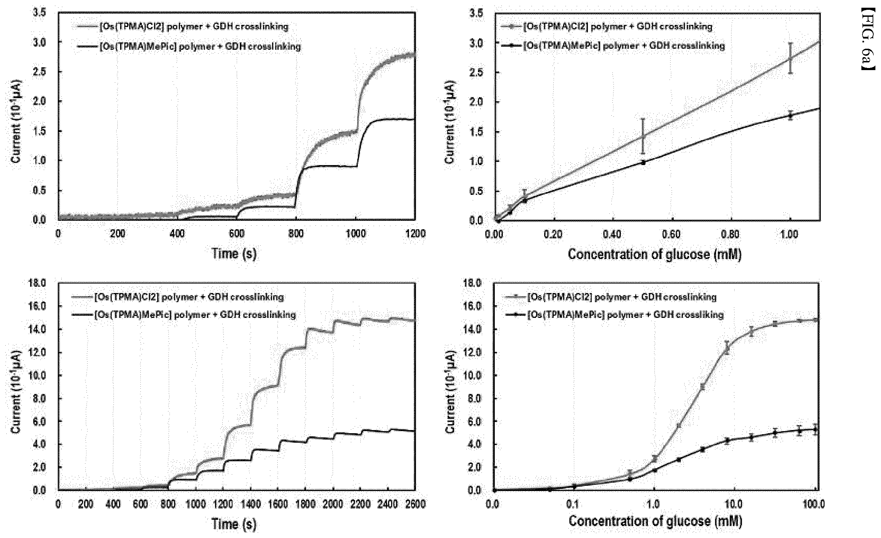

While stirring the electrode in which the oxidation-reduction polymer and glucose dehydrogenase were immobilized, prepared in Example 2-1, at 150 rpm after adding a stir bar to 50 mL of 10 mM PBS solution, an i-t curve was measured. A certain amount of the solution in which glucose was dissolved in 10 mM PBS was added per 200 seconds and a current was measured under the concentration of 0.01, 0.05, 0.1, 0.5, 1, 2, 4, 8, 16, 32, 64, 100 mM to confirm the change in current. The result was shown in FIG. 6a.

As a result of the test, the current was gradually increased as the glucose concentration gradually increased in the electrodes in which the oxidation-reduction polymers Nos. 37 and 38 were crosslinked with the enzyme, respectively. First, in the current-time graph, both electrodes showed a stepwise increase up to the concentration of 100 mM, and the change was evident even in the low concentration section (0 ~ 1 mM). In addition, when the current generated according to the concentration was averaged and a current-concentration graph was plotted, correlation with the magnitude of the current generated by each glucose concentration was confirmed. In the low concentration section, the amount of constant current increased linearly, but as the concentration gradually increased, in the high concentration section of 16 mM or more, the current increase was gradually reduced, showing that the current value appeared constant. It could be seen that this reached the maximum value at which glucose could be oxidized in an electrode, and the current increase was gradually reduced and it reached the limiting catalytic current (iₘₐₓ). Furthermore, the electrode crosslinked with the oxidation-reduction polymer No. **37** showed a maximum current of about 1.5 µA, whereas the electrode crosslinked with the oxidation-reduction polymer No. **38** showed a maximum current of about 0.5 µA, 1/3 lower than that of the **37**-based electrode.

While stirring the electrode in which the oxidation-reduction polymer and glucose dehydrogenase were immobilized, prepared in Example 2-1, at 150 rpm after adding a stir bar to 50 mL of 10 mM PBS solution, an i-t curve was measured. In the electrodes in which the oxidation-reduction polymers 1 and 2 comprising a transition metal complex comprising a C-N ligand were crosslinked with an enzyme, a certain amount of the solution in which glucose was dissolved in 10 mM PBS was added per 200 seconds and a current was measured under a low concentration section increasing to 0.01, 0.05, 0.1, 0.5 mM and under a high concentration section increasing to 1, 5, 10, 50, 100 mM, respectively, to confirm the change in current. The result was shown in FIGs. 6b to 6c.

As a result of the test, the current was gradually increased stepwise in both electrodes in the low concentration section (0 ~ 0.5 mM) and high concentration section (0 ~ 100 mM). In the low concentration section, the amount of constant current increased linearly, while in the high concentration section, the concentration gradually increased, showing that the current value appeared constant from a concentration of 50 mM or more. This reached the maximum value at which glucose could be oxidized in the electrodes, and the electrode in which the oxidation-reduction polymer 1 comprising a transition metal complex comprising a C-N ligand was crosslinked showed a maximum current of about 1.0 µA, and the oxidation-reduction polymer 2 comprising a transition metal complex comprising a C-N ligand was crosslinked showed a maximum current of about 0.2 µA.

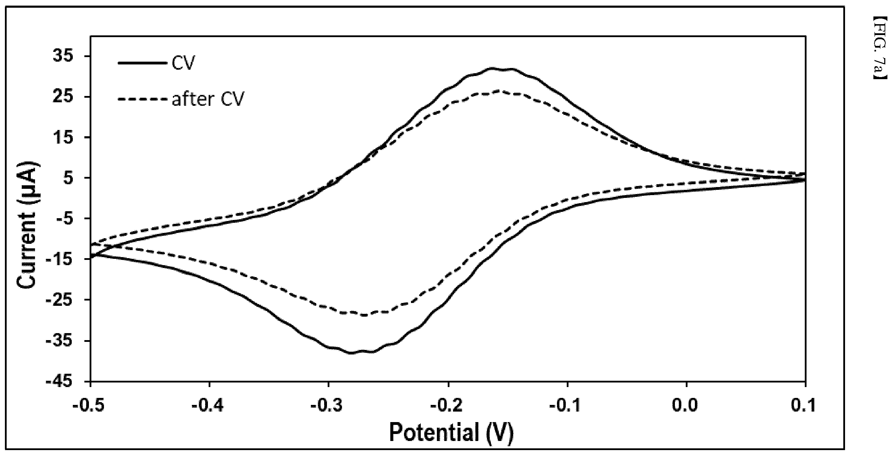

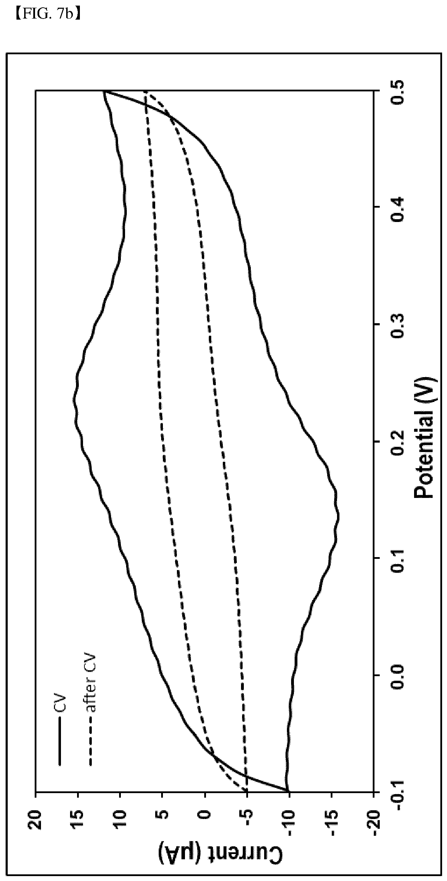

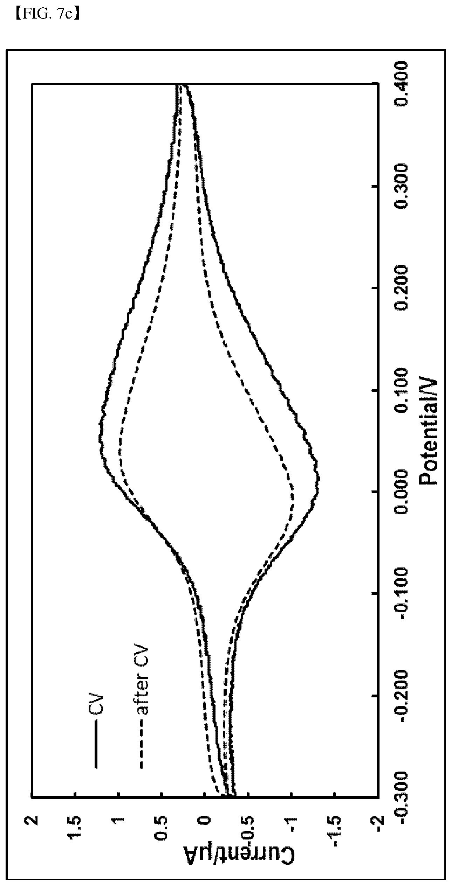

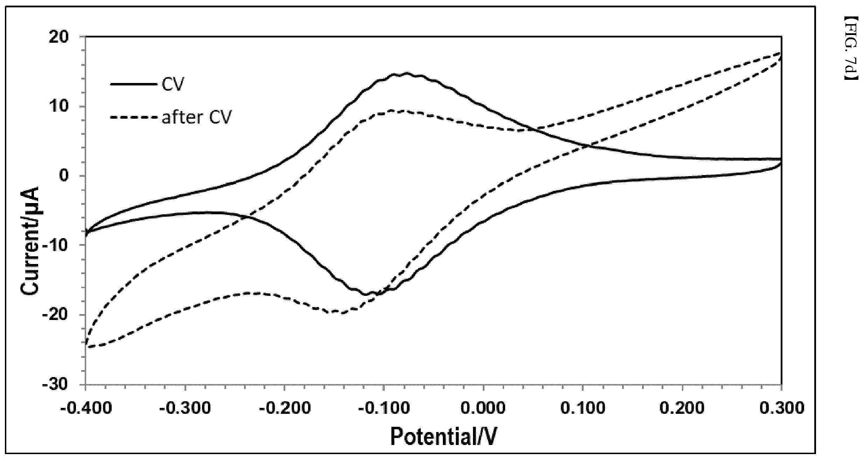

Furthermore, the change in current before and after the cyclic voltammetry test was measured. Specifically, for the change in current, it was measured at -0.5 to +0.1 V in 10 mM PBS, and at -0.3 to +0.4 V in case of the oxidation-reduction polymers 1 and 2 comprising an electron transfer mediator comprising a C-N ligand and PFPA, and the result was shown in FIGs. 7a to 7d. In FIGs. 7a to 7d, CV represents that measured immediately after completing the electrode, and after CV represents a value measured after stirring for 1 hour in a PBS solution.

As above, the oxidation-reduction polymer comprising PFPA was well immobilized to the enzyme and the electrode surface and showed clear response even in the low concentration section, and a linear current change could be confirmed even at a certain concentration. In addition, the crosslinked electrodes exhibited the limiting catalytic current of 1.5 µA, 0.5 µA, 1.0 µA, 0.2 µA, respectively. Through this, it was demonstrated that the immobilization method using a TPMA-osmium complex-based electron transfer mediator or C-N ligand-osmium complex-based electron transfer mediator and a PFPA polymer matrix could function as a blood glucose sensor.

## Claims

1. A polypentafluorophenyl ester-based polymer for manufacturing an oxidation-reduction polymer material.

2. The polymer for manufacturing an oxidation-reduction polymer material according to claim 1, which is prepared by introducing primary and secondary amine-based compounds to the polypentafluorophenyl ester through aminolysis.

3. The polymer for manufacturing an oxidation-reduction polymer material according to claim 1, wherein the polymer comprises a polymer selected from the group consisting of a polypentafluorophenyl acrylate (PPFPA) homopolymer, a polypentafluorophenyl methacrylate (PPFPM) homopolymer, a polypentafluorophenyl acrylate-polydimethylacrylamide (PPFPA-PDMA) copolymer, a polypentafluorophenyl methacrylate-polydimethylacrylamide (PPFPM-PDMA) copolymer, a polypentafluorophenyl acrylate-polyacrylamide (PPFPA-PAA) copolymer, a polypentafluorophenyl methacrylate-polyacrylamide (PPFPM-PAA) copolymer.

4. The polymer for manufacturing an oxidation-reduction polymer material according to claim 1, having a structure of Chemical formula 1 or 2 below: 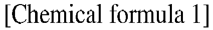 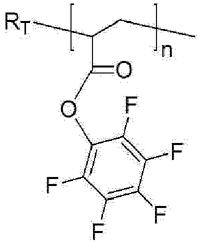 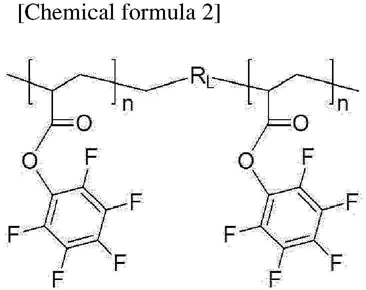 in the Chemical formula 1 or 2,
R_{T} and R_{L} are each independently selected from the group consisting of a substituted or non-substituted alkylene group having 1 to 20 carbon atoms, a substituted or non-substituted cycloalkylene group having 1 to 20 carbon atoms, a substituted or non-substituted ethylene glycol group having 3 to 30 carbon atoms, a substituted or non-substituted arylene group having 6 to 30 carbon atoms, a substituted or non-substituted heteroarylene group having 3 to 30 carbon atoms, a substituted or non-substituted alkenyl group having 2 to 40 carbon atoms and a substituted or non-substituted alkynyl group having 2 to 40 carbon atoms; and
the n is an integer of 10 to 300.

5. The polymer for manufacturing an oxidation-reduction polymer material according to claim 4, wherein the R_{T} and R_{L} are each independently selected from the group consisting of polydimethylacrylamide (PDMA), polyacrylamide (PAA), polystyrene (PS), polyethylene glycol (PEG), polyethyleneoxide (PEO), polymethylmethacrylate (PMMA), polyvinylimidazole (PVI), polyvinylpyridine (PVP) and polysiloxane (PDMS) with a molecular weight of 1,000 g/mol ~ 50,000 g/mol.

6. The polymer for manufacturing an oxidation-reduction polymer material according to claim 1, having a weight average molecular weight within a range of 1,000 g/mol to 500,000 g/mol.

7. An oxidation-reduction polymer material for an electrochemical sensor comprising the polymer according to any one claim of claim 1 to claim 6.

8. The oxidation-reduction polymer material according to claim 7, wherein a functional group selected from the group consisting of primary and secondary amine groups and ammonium groups and a transition metal complex are combined to the polymer.

9. The oxidation-reduction polymer material according to claim 8, wherein the transition metal complex has a structure of Chemical formula 3 or 4 below: 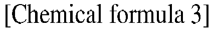 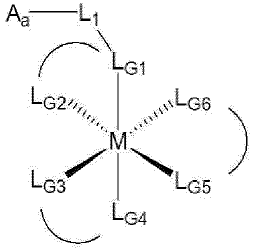 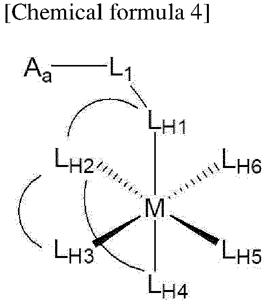 in the Chemical formula 3 or 4,
M is one kind of transition metals selected from the group consisting of Os, Rh, Ru, Ir, Fe and Co; and
in the Chemical formula 3, L_{G1} and L_{G2} are combined with each other to form a bidentate ligand selected from Chemical formula 5 to 6 below; and
L_{G3} and L_{G4} are combined with each other to form a bidentate ligand selected from Chemical formula 5 to 6 below; and L_{G5} and L_{G6} are combined with each other to form a bidentate ligand selected from Chemical formula 5 to 6 below; and 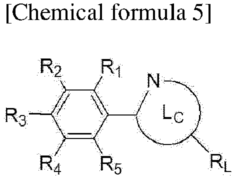 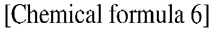 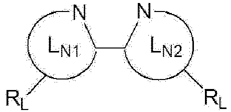
in the Chemical formula 5, Lc is a heterocyclic compound comprising one or more nitrogen atoms, and is linked with the chemical formula of benzene at position 2; and
in the Chemical formula 6, L_{N} is a heterocyclic compound comprising one or more nitrogen atoms, and L_{N1} and L_{N2} are linked to each other at position 2, respectively; and
R_{L} is all functional groups of heterocyclic compounds Lc, L_{N1} and L_{N2}; and
the R₁, R₂, R₃, R₄, R₅ and R_{L} are each independently selected from the group consisting of a substituted or non-substituted alkyl group having 1 to 10 carbon atoms, a substituted or non-substituted ethylene glycol group having 2 to 20 carbon atoms, a substituted or non-substituted alcohol group having 1 to 20 carbon atoms, a substituted or non-substituted alkyl halogen group having 1 to 20 carbon atoms, a substituted or non-substituted thiol group having 1 to 20 carbon atoms, a substituted or non-substituted alkyl azide group having 3 to 20 carbon atoms, a substituted or non-substituted aryl azide group having 7 to 30 carbon atoms, a substituted or non-substituted alkenyl group having 2 to 40 carbon atoms, a substituted or non-substituted alkynyl group having 2 to 40 carbon atoms, a cyano group, a halogen group, heavy hydrogen and hydrogen; and
L_{H1}, L_{H3} and L_{H4} of the Chemical formula 4 are combined with each other centering on L_{H2} to form a tetradentate ligand represented by Chemical formula 7 below; and
then, L_{H5} and L_{H6} form a monodentate ligand for transition metal M as represented in Chemical formula 8, respectively; or L_{H5} and L_{H6} are combined with each other to form a bidentate ligand for transition metal M as represented by Chemical formula 9 below, respectively; and 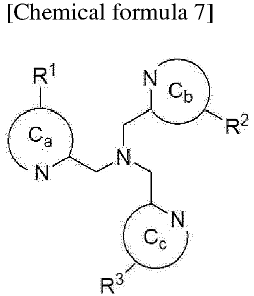
in the Chemical formula 7,
Cₐ, C_{b} and C_{c} are heterocyclic compounds comprising one or more nitrogen atoms, and the heterocyclic compounds are linked to an amine group and a methylene group at position 2, and 3 nitrogens of the three hetero rings, and 1 nitrogen at the center connecting the three hetero rings to each other are linked to transition metal M, and R₁, R₂, R₃ are all functional groups of Cₐ, C_{b}, C_{c}, and the R₁, R₂ and R₃ are each independently selected from the group consisting of a substituted or non-substituted alkyl group having 1 to 10 carbon atoms, a substituted or non-substituted ethylene glycol groups having 2 to 20 carbon atoms, a substituted or non-substituted alcohol group having 1 to 20 carbon atoms, a substituted or non-substituted alkyl halogen group having 1 to 20 carbon atoms, a substituted or non-substituted thiol group having 1 to 20 carbon atoms, a substituted or non-substituted alkyl azide group having 3 to 20 carbon atoms, a substituted or non-substituted aryl azide group having 7 to 30 carbon atoms, a substituted or non-substituted alkenyl group having 2 to 40 carbon atoms, a substituted or non-substituted alkylnyl group having 2 to 40 carbon atoms, a cyano group, a halogen group, heavy hydrogen and hydrogen; 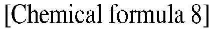 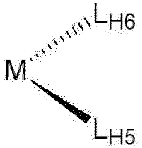 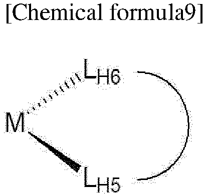 in the Chemical formula 8,
L_{H5} and L_{H6} are independently a monodentate ligand, and are each independently a heterocyclic compound comprising one or more -H, -F, -Cl, -Br, -I, -NO₂, -NCCH₃, -CO, -OH₂, -NH₃ or
nitrogen atoms; and
in the Chemical formula 9,
L_{H5}-L_{H6} are bidentate ligands, and are catechol, acetylacetone, 2-picolinic acid, 2-pyridinecarboxamide, 2,2-bipyridine, or 2,2-bithiazole; and
the L₁ is independently selected from the group consisting of a substituted or non-substituted alkylene group having 1 to 20 carbon atoms, a substituted or non-substituted cycloalkylene group having 1 to 20 carbon atoms, a substituted or non-substituted ethylene glycol group having 2 to 30 carbon atoms, a substituted or non-substituted arylene group having 6 to 30 carbon atoms, and a substituted or non-substituted heteroarylene group having 3 to 30 carbon atoms; and
the Aₐ is selected from the group consisting of an amine group and ammonium group.

10. A method for preparation of an oxidation-reduction polymer material thin film, comprising coating the oxidation-reduction polymer material according to claim 7 to an electrode, and then hardening the coated electrode.

11. An oxidation-reduction polymer material thin film prepared by the method for preparation according to claim 10.

12. An electrochemical biosensor comprising the oxidation-reduction polymer material thin film prepared by the method for preparation according to claim 10.

13. A sensing layer for an electrochemical biosensor comprising an enzyme capable of redoxing a liquid biological sample; and
the electron transfer mediator according to claim 7.

14. The sensing layer for an electrochemical biosensor according to claim 13, wherein the enzyme comprises
one or more kinds of oxidoreductases selected from the group consisting of dehydrogenase, oxidase and esterase; or
one or more kinds of oxidoreductases selected from the group consisting of dehydrogenase, oxidase and esterase, and one or more kinds of cofactors selected from the group consisting of flavin adenine dinucleotide (FAD), nicotinamide adenine dinucleotide (NAD), and pyrroloquinoline quinone (PQQ).

15. An electrochemical biosensor comprising the sensing layer for an electrochemical biosensor according to claim 14.

16. The electrochemical biosensor according to claim 15, wherein the sensor is a continuous blood glucose monitoring sensor.

17. A device comprising the polymer for manufacturing an oxidation-reduction polymer material according to any one claim of claim 1 to claim 6.

18. The device according to claim 17, wherein the device is an electrochemical biosensor.

19. The device according to claim 18, wherein the device is an implantable device.
